# EUROPEAN PATENT SPECIFICATION

(11) **EP 3 632 312 B1**
(45) Date of publication and mention of the grant of the patent: **24.05.2023**
(21) Application number: 18808879.3
(22) Date of filing: 26.04.2018
(51) Int. Cl.: A61B 5/15, G01N 33/49, G01N 33/487, A61B 5/155, G01N 35/00

(54) **INTEGRATED STRIP CARTRIDGE FOR GLUCOSE METER**
INTEGRIERTE BANDKASSETTE FÜR GLUKOSEMESSER
CARTOUCHE DE BANDELETTES INTÉGRÉE POUR GLUCOMÈTRE

(30) Priority: 02.06.2017 KR 20170068965
(43) Date of publication of application: 08.04.2020
(73) Proprietor: I-sens, Inc., Seoul 06646 (KR)
(72) Inventor: CHAE, Kyung Chul, Seongnam-si, Gyeonggi-do 13589 (KR); CHOI, Hyun Ho, Seoul 03097 (KR); RYU, Goang Yel, Goyang-si, Gyeonggi-do 10518 (KR); WANG, Ji Hoon, Goyang-si, Gyeonggi-do 10240 (KR); LEE, Jin Won, Seoul 04724 (KR); CHA, Geun Sig, Seoul 03610 (KR); NAM, Hak Hyun, Seoul 01227 (KR)
(74) Representative: Sander, Rolf
(86) International application number: PCT/KR2018/004831
(87) International publication number: WO 2018/221862

(56) References cited:
- EP-A1- 1 321 769
- WO-A1-2012/177908
- WO-A1-2013/180804
- WO-A2-02/08753
- JP-A- 2010 504 844
- KR-A- 20070 073 914
- KR-A- 20110 095 197
- US-A1- 2003 191 415
- US-A1- 2008 299 009
- US-A1- 2008 299 009

## Description

### TECHNICAL FIELD

The present invention relates to a strip cartridge for an integrated glucose meter. More particularly, the present invention relates to a strip cartridge for an integrated glucose meter, in which catch holders able to hold test strips is provided on the top end portion of an internal container, such that test strips can be accurately and easily stacked and stored in the internal space of the internal container, and the internal container having test strips stacked and stored therein can be inserted into a main container, so that a fabrication process can be conveniently and rapidly performed. In addition, errors, such as misalignment or overturning of the stacking and arrangement of test strips, which may occur during the storage of the test strips, can be prevented, thereby enabling the fabrication process to be performed more quickly and accurately.

### BACKGROUND ART

Diabetes or diabetes mellitus is a chronic medical condition that is common in modern people. In the Republic of Korea, it affects 2 million people, about 5% of the total population.

Diabetes is caused from the absolute deficiency or relative insufficiency of insulin produced by the pancreas, due to various causes such as obesity, stress, poor eating habits, inherited hereditary factors, in which glucose balance in the blood may be disturbed, thereby resulting in a high blood sugar level.

The blood usually contains a certain concentration of glucose, and tissue cells gain energy from the glucose.

However, when the content of glucose is increased excessively, it is not properly stored in the liver, muscle, or adipose tissue and accumulates in the blood. As a result, patients with diabetes maintain a much higher blood glucose level than other people. Excessive blood glucose passes through the tissues and is discharged into the urine, resulting in deficiency of glucose, which is absolutely necessary for all tissues of the body, thereby causing abnormalities in respective body tissues.

Diabetes mellitus is characterized by substantial absence of subjective symptoms at the beginning of the condition. When diabetes mellitus progresses, diabetes-specific symptoms such as diarrhea, polyuria, weight loss, general anxiety, skin itchiness, and scarring of the hands and feet are present. Further progression of diabetes leads to complications such as visual disturbances, hypertension, kidney disease, paralysis, periodontal disease, muscle spasms and neuralgia, as well as gangrene.

Systematic blood glucose measurement and treatment should be performed to diagnose diabetes beforehand and manage the condition to prevent the progression of diabetes into complications associated therewith.

For people with diabetes or people having higher than normal blood glucose, even though diabetes has not yet developed, medical device manufacturers offer a variety of blood glucose meters to measure blood glucose at home.

In such a blood glucose meter, a test strip is input to a container of the blood glucose meter, a blood sample is absorbed by the input test strip, a glucose level is measured by inspecting the absorbed blood sample using a glucose measuring module within the container, and a measurement result is calculated by a calculator and is output on a separate display.

To use such a blood glucose meter, a user should possess and carry a separate strip storage container in which a plurality of test strips are stored. A glucose measuring process is performed by taking one test strip from the strip storage container and inputting the test strip to the blood glucose meter, and after the glucose measuring process, the used test strip must be discharged and discarded, thereby making it inconvenient to use the blood glucose meter. In addition, since the strip storage container must be carried separately, there are significant difficulties in the use of the blood glucose meter.

To reduce or overcome such inconveniences in the use thereof, various types of integrated blood glucose meters have recently been developed, in each of which a strip cartridge storing a plurality of test strips can be accommodated in an internal space of the blood glucose meter, and the test strips can be supplied individually from the strip cartridge.

For such an integrated glucose meter, a strip cartridge is essential. The strip cartridge is fabricated such that a plurality of test strips are stored in the strip cartridge. A process of storing a plurality of test strips in a strip cartridge is generally performed as an automated process. In the process of stacking and storing the plurality of test strips, problems may frequently occur. For example, the test strips in a stacked and arranged state may be misaligned or overturned. Accordingly, a range of difficulties may be caused in fabrication. For example, a defect rate may be increased, and a fabrication process may be stopped. WO02/08753 discloses a prior art test device for testing fluid concentrations.

### DISCLOSURE

### Technical Problem

Accordingly, the present invention has been made in consideration of the above-described problems occurring in the related art, and an objective of the present invention is to provide a strip cartridge for an integrated glucose meter, in which errors, such as misalignment or overturning of the stacking and arrangement of test strips, which may occur during the storage of test strips in the cartridge, can be prevented, thereby enabling a fabrication process to be performed more quickly and accurately.

Another objective of the present invention is to provide a strip cartridge for an integrated glucose meter, in which catch holders able to hold test strips are provided on the top end portion of an internal container, such that test strips can be accurately and easily stacked and stored in the internal space of the internal container, and the internal container having test strips stacked and stored therein can be inserted into a main container, so that the fabrication process can be conveniently and rapidly performed.

Another objective of the present invention is to provide a strip cartridge for an integrated glucose meter, in which, in a process of loading a test strip stacked and stored within the cartridge to an uppermost layer, the test strip can be guided to a designated position, so that the supply of the test strip to be used in the integrated glucose meter can be accurately performed.

### Technical Solution

According to an aspect of the present invention, provided is a strip cartridge in accordance with independent claim 1 for an integrated blood glucose meter. The strip cartridge is insertable into the integrated glucose meter and is configured such that a plurality of test strips are stored therein. The strip cartridge may include: a vessel-shaped main container having an open bottom end portion and a strip supply port provided on a top end portion, the strip supply port allowing test strips to be discharged individually therethrough; an internal container shaped as a rectangular pipe allowing test strips to be stored and stacked on each other in a top-bottom direction in an internal space of the internal container, wherein the internal container is inserted into the main container and includes catch holders on a top end portion thereof to prevent test strips from being dislodged upwardly; a pressure loading module coupled to a bottom end portion of the internal container and elastically pressing test strips, stacked in the internal space of the internal container, in a direction from bottom to top.

Here, the catch holders are configured such that an uppermost test strip caught and held by the catch holders is loaded in the strip supply port.

In addition, the catch holders include: elastic bodies protruding upwardly from opposite edges of the top end portion of the internal container to face each other, and being elastically deformable in a direction toward or away from each other; and catch protrusions protrude from top end portions of the elastic bodies, respectively, in a direction in which the catch protrusions face each other. Test strips are caught and held by the catch protrusions when test strips located within the internal container are pressed upwardly by the pressure loading module.

In addition, the catch protrusions may have horizontal bottom surfaces, by which test strips are caught and held, and inclined top surfaces inclined downwardly in a direction in which the catch protrusions face each other.

In addition, the elastic bodies may have inclined surfaces facing each other, the inclined surfaces being inclined toward each other in a direction from bottom to top, such that test strips are held and guided without movement.

In addition, the strip supply port may include: a protruding body protruding upwardly from the top end portion of the main container, enabling a single test strip to be loaded in an internal space of a protruding area; and horizontal holes provided in opposite ends of the protruding body, enabling the single test strip to be slid and discharged by being pressed by the separate discharge/supply means. An intermediate opening may be provided in an intermediate portion of the protruding body, such that the catch holders of the internal container are disposed in the intermediate portion of the protruding body.

In addition, among the horizontal holes provided in the opposite ends of the protruding body, a width of the horizontal hole, through which test strips are discharged, may be greater than a width of test strips, and a width of the remaining horizontal hole may be smaller than the width of test strips.

In addition, the pressure loading module may include: a base body coupled to the main container to close the open bottom end portion of the main container; a guide rod coupled to the base body to protrude upwardly so as to be inserted into the internal container; a pressing body coupled to the guide rod to be movable linearly in a top-bottom direction; and an elastic spring applying upward elastic force to the pressing body so that test strips in the internal space of the internal container are elastically pressed upward.

In addition, a fixing guide for fixing the position of the main container inserted into the integrated glucose meter may be provided on an outer surface portion of the main container.

### Advantageous Effects

According to the invention, errors, such as misalignment or overturning of the stacking and arrangement of test strips, which may occur during the storage of test strips in the cartridge, can be prevented, thereby enabling a fabrication process to be performed more quickly and accurately.

In addition, the catch holders able to hold test strips are provided on the top end portion of the internal container, such that test strips can be accurately and easily stacked and stored in the internal space of the internal container, and the internal container having test strips stacked and stored therein can be inserted into the main container, so that the fabrication process can be conveniently and rapidly performed.

In addition, in a process of loading a test strip stacked and stored within the cartridge to an uppermost layer, the test strip can be guided to a designated position, so that the supply of the test strip to be used in the integrated glucose meter can be accurately performed.

### DESCRIPTION OF DRAWINGS

FIG. 1 is a perspective view schematically illustrating an external shape of a strip cartridge for an integrated blood glucose meter according to an embodiment of the invention;
FIG. 2 is an exploded perspective view schematically illustrating a configuration of the strip cartridge for an integrated blood glucose meter according to the embodiment of the invention;
FIG. 3 is a cross-sectional view schematically illustrating an internal structure of the strip cartridge for an integrated blood glucose meter according to the embodiment of the invention;
FIG. 4 is another cross-sectional view taken along line A-A in FIG. 3;
FIG. 5 is another cross-sectional view schematically illustrating an internal structure of the strip cartridge for an integrated blood glucose meter according to the embodiment of the disclosure, taken in a further direction; and
FIG. 6 is a view illustrating a structure of storing and stacking test strips in the strip cartridge for an integrated blood glucose meter according to the embodiment of the invention.

### MODE FOR INVENTION

Hereinafter, embodiments of the present invention will be described in detail with reference to the accompanying drawings. Throughout this document, reference should be made to the drawings, in which the same reference numerals and symbols will be used to designate the same or like components. In the following description of the present disclosure, detailed descriptions of known functions and components incorporated herein will be omitted in the case that the subject matter of the present disclosure may be rendered unclear thereby.

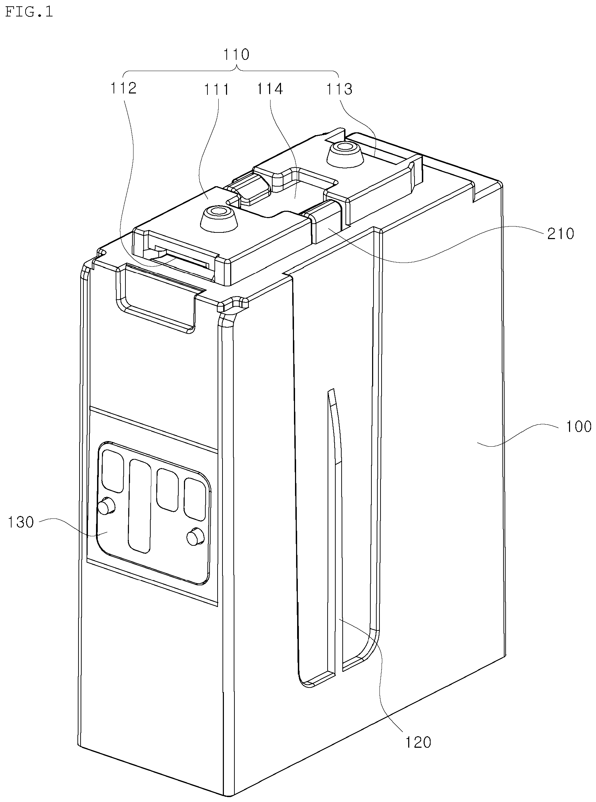

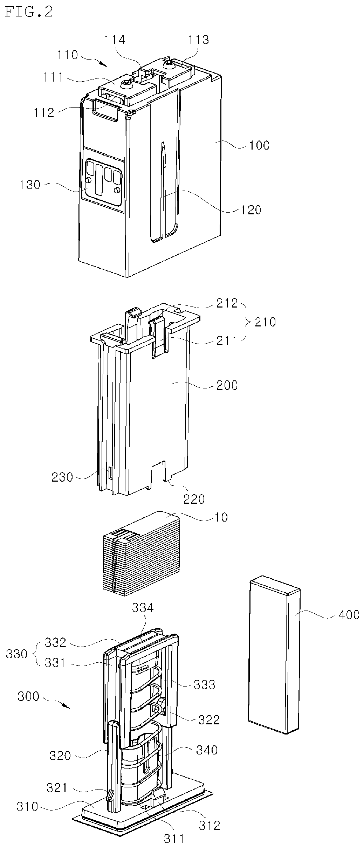

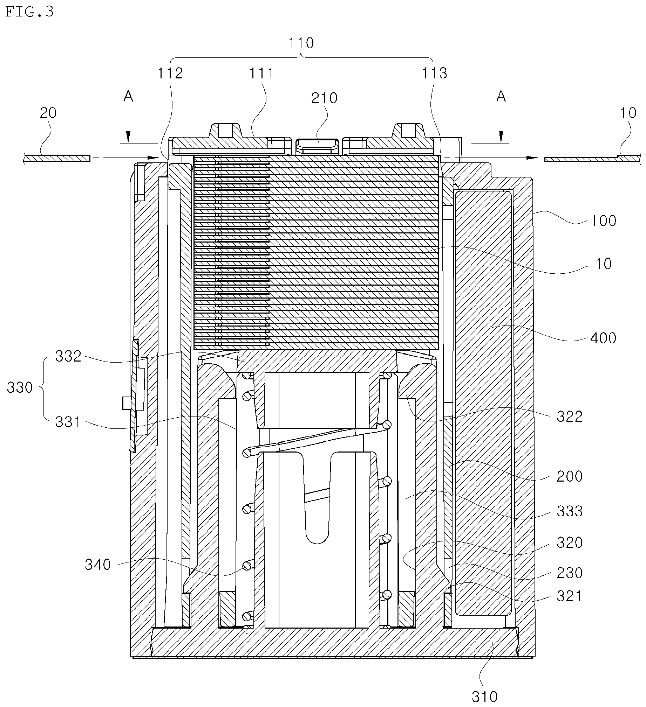

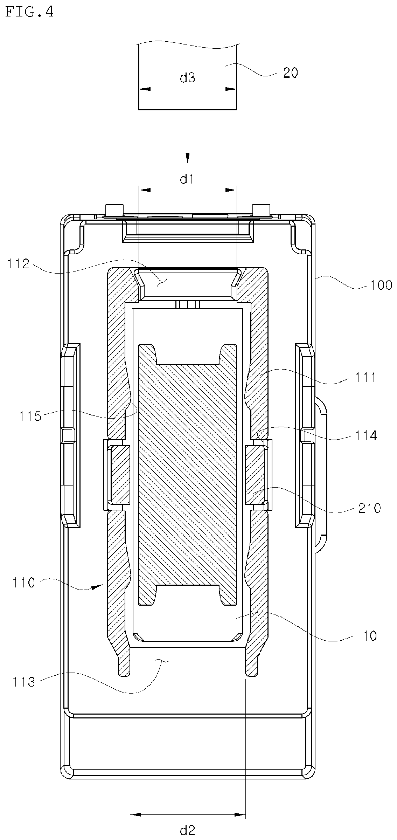

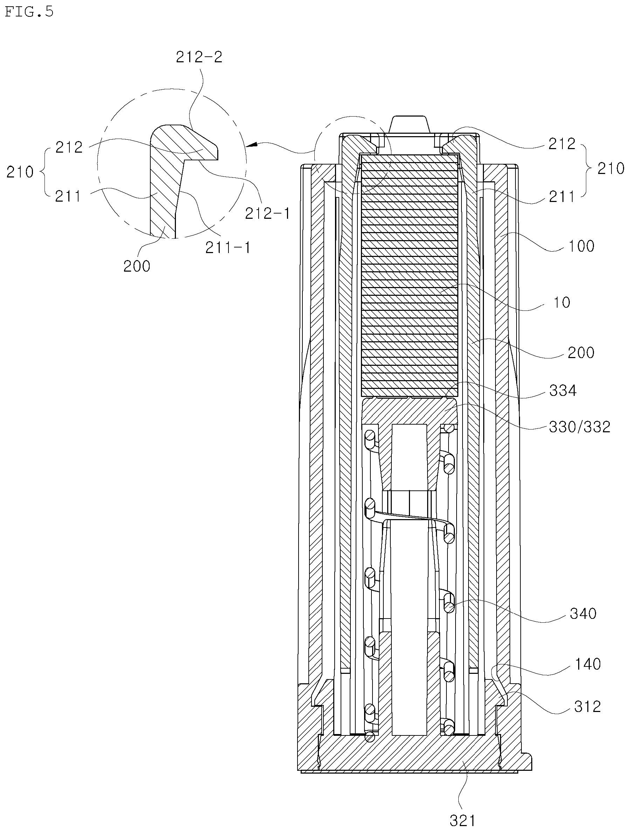

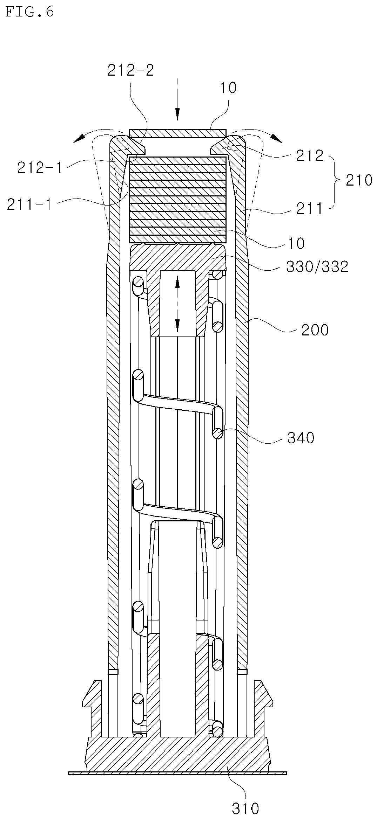

FIG. 1 is a perspective view schematically illustrating an external shape of a strip cartridge for an integrated blood glucose meter according to an embodiment of the invention, FIG. 2 is an exploded perspective view schematically illustrating a configuration of the strip cartridge for an integrated blood glucose meter according to the embodiment of the invention, FIG. 3 is a cross-sectional view schematically illustrating an internal structure of the strip cartridge for an integrated blood glucose meter according to the embodiment of the invention, FIG. 4 is another cross-sectional view taken along line A-A in FIG. 3, FIG. 5 is another cross-sectional view schematically illustrating an internal structure of the strip cartridge for an integrated blood glucose meter according to the embodiment of the invention, taken in a further direction, and FIG. 6 is a view illustrating a structure of storing and stacking test strips in the strip cartridge for an integrated blood glucose meter according to the embodiment of the invention.

The strip cartridge for an integrated blood glucose meter according to the embodiment of the invention is configured to store a plurality of test strips 10 therein and to be accommodated in the integrated glucose meter. The strip cartridge includes a main container 100, an internal container 200, and a pressure loading module 300.

The main container 100 provides an overall external structure of the strip cartridge. The main container 100 has the shape of a rectangular vessel with the bottom end portion thereof being open. The main container 100 has a strip supply port 110 on the top end portion thereof, the strip supply port 110 allowing the test strips to be discharged and supplied individually therethrough.

A cartridge detector 130 storing unique identification information of a corresponding strip cartridge may be attached to an outer surface portion of the main container 100. The cartridge detector 130 may be implemented as an electronic chip, such that information regarding the strip cartridge, for example, information regarding the number of the test strips 10 stored in the strip cartridge is stored therein. Thus, when the strip cartridge is inserted into the integrated glucose meter, the strip cartridge and the integrated glucose meter can transmit information to and receive information from each other via the cartridge detector 130. Various pieces of information regarding the strip cartridge can be transmitted to the integrated glucose meter, thereby facilitating the use and management of the strip cartridge.

In addition, a fixing guide 120 for fixing the position of the main container 100 inserted into the integrated glucose meter may be provided on an outer surface portion of the main container 100. That is, the integrated glucose meter has an insertion port, through which the strip cartridge is inserted. When the insertion of the strip cartridge according to the embodiment of the invention into the insertion port of the integrated glucose meter is completed, the strip cartridge is required to fixedly remain at the inserted position without movement. In this regard, the fixing guide 120 may be provided on the outer surface portion of the main container 100. The fixing guide 120 may have a variety of shapes. For example, the fixing guide 120 may have the shape of a wedge inclined in the direction in which the main container 100 is inserted.

The internal container 200 has the shape of a rectangular pipe such that the plurality of test strips 10 are stored and stacked in the internal space thereof, and is inserted into the main container 100. Catch holders 210 are provided on the top end portion of the internal container 200 to catch and hold an uppermost test strip 10 among the test strips 10 to prevent the test strips 10 from being dislodged upwardly. A guide rail (not shown) and a guide groove (not shown) having corresponding shapes may be provided on an outer surface portion of the internal container 200 and an inner surface portion of the main container 100 to guide a path on which the internal container 200 is inserted into the main container 100. Here, each of the guide rail and the guide groove may be shaped to be asymmetrical in the lateral direction, such that the internal container 200 can only be inserted in a designated direction but cannot be inserted in the opposite direction.

The pressure loading module 300 is coupled to a bottom end portion of the internal container 200, and is configured to elastically press the plurality of test strips 10, stored and stacked within the internal container 200, upward.

According to this structure, the plurality of test strips 10 stacked and stored within the internal container 200 are elastically pressed upward within the internal container 200 by the pressure loading module 300, so that the uppermost test strip 10 among the test strips 10, stored and stacked within the internal container 200, is caught and held by the catch holders 210 provided on the top end portion of the internal container 200. Consequently, the plurality of test strips 10 are reliably held and maintained in the stacked and arranged state.

Here, the catch holders 210 are configured such that the uppermost test strip 10 caught and held by the catch holders 210 is loaded in the strip supply port 110 of the main container 100. Thus, the uppermost test strip 10 among the plurality of test strips 10 is loaded in the strip supply port 110 while being caught and held by the catch holders 210. In this state, when a discharge/supply means 20 (see FIG. 4) separately provided inside of the integrated glucose meter moves linearly through the strip supply port 110, the test strip 10 located in the upmost position of the internal container 200 is pressed by the discharge/supply means 20 to be moved and supplied to a separate strip outlet (not shown). In this state in which the test strip 10 is supplied and discharged through the strip outlet, a glucose measurement is performed by absorbing blood by the test strip 10.

Next, a detailed configuration of the strip cartridge according to the embodiment of the invention will be described in more detail.

First, the pressure loading module 300 includes a base body 310, guide rods 320, a pressing body 330, and an elastic spring 340. The base body 310 is coupled to the main container 100 to close the open bottom end portion of the main container 100. The guide rods 320 are coupled to the base body 310 to protrude upwardly so as to be inserted into the internal container 200. The pressing body 330 is coupled to the guide rods 320 to be movable linearly in the top-bottom direction, thereby pressing and supporting the test strips 10. The elastic spring 340 applies upward elastic force to the pressing body 330 so that the test strips 10 in the internal space of the internal container 200 are elastically pressed upward.

The pressing body 330 includes a pressing/supporting portion 332 having the shape of a flat plate to press and support the test strips 10 and guides 331 provided on opposite end portions of the pressing/supporting portion 332 to extend in the top-bottom direction. A plurality of projections 334 may be provided on the top surface of the pressing/supporting portion 332 and extend in a linear direction to reduce a surface area to be in contact with the corresponding test strip 10. The elastic spring 340 applies upward elastic force to the pressing body 330. Here, the guides 331 respectively have a guide slit 333 extending in the top-bottom direction, the guide slit 333 being able to restrict a top-bottom movement path of the pressing body 330. The guide rods 320 may respectively have a stopper protrusion 322 able to be fitted into and engaged with the corresponding guide slit 333.

In addition, the guide rod 320 of the pressing body 330 has a catch hook 321 protruding from an outer surface portion thereof. A catch hole 230 is provided in a side portion of the internal container 200, in a position corresponding to the catch hook 321. As the catch hook 321 is fitted into and engaged with the catch hole 230, the pressure loading module 300 is fixedly coupled to the internal container 200. In addition, coupling protrusions 220 are provided on the bottom end portion of the internal container 200, and coupling holes 311 are provided on the top portion of the base body 310. The coupling protrusions 220 are fitted into and engaged with the coupling holes 311, thereby fixedly coupling the internal container 200 and the pressure loading module 300.

In addition, separate catch protrusions 312 may protrude upwardly from the top surface of the base body 310, and the main container 100 may have coupling recesses 140 in inner surface portions thereof, such that the catch protrusions 312 may be fitted into and engaged with the coupling recesses 140. As the catch protrusions 312 are fitted into and engaged with the coupling recesses 140, the pressure loading module 300 is coupled to the main container 100.

The strip supply port 110 of the main container 100 includes a protruding body 111 protruding upwardly from the top end portion of the main container 100, such that a single test strip 10 can be loaded in an internal space of a protruding area. Horizontal holes 112 and 113 are provided in opposite ends of the protruding body 111, such that the single test strip 10 can be slid and discharged by being pressed by the separate discharge/supply means 20. An intermediate opening 114 is formed in an intermediate portion of the protruding body 111, such that the catch holders 210 of the internal container 200 are disposed in the intermediate portion of the protruding body 111.

Here, among the horizontal holes 112 and 113 formed in the opposite ends of the protruding body 111, the width d2 of the horizontal hole 113, through which the test strips 10 are discharged, is set to be greater than the width d of the test strips 10, as illustrated in FIG. 4. The width d1 of the remaining horizontal hole 112 is set to be smaller than the width d of the test strips 10. The discharge/supply means 20 has the shape of a flat plate to move through the horizontal holes 112 and 113 of the strip supply port 110. In this process, the discharge/supply means 20 pushes and supplies the uppermost test strip 10 outwardly. Here, the width d3 of the discharge/supply means 20 shall be set to be smaller than or equal to the width d1 of the horizontal hole 112.

This structure can prevent any possibility that the test strips 10 may be discharged reversely from the internal space of the protruding body 111 through the inlet-side horizontal hole 112. Accordingly, the test strips 10 can be more accurately supplied.

In addition, guide protrusions 115 may protrude inwardly from inner surface portions of the protruding body 111 to guide the test strips 10 to the designated position. Thus, even in the case in which the arranged state of the test strips 10, stacked within the internal container 200 in the top direction, is slightly misaligned, the test strips 10 can be guided to the designated position by the guide protrusions 115 while being moved upwardly into the internal space of the protruding body 111. Accordingly, the operation of supplying the test strips 10 by the discharge/supply means 20 can be more accurately performed.

In addition, the catch holders 210 provided on the top end portion of the internal container 200 include elastic bodies 211 protruding upwardly from opposite edges of the top end portion of the internal container 200 to face each other. The elastic bodies 211 are elastically deformable in a direction toward or away from each other. Catch protrusions 212 protrude from the top end portions of the elastic bodies 211, respectively, in a direction in which the catch protrusions 212 face each other. The test strips 10 are caught and held by the catch protrusions 212 when the test strips 10 located within the internal container 200 are pressed upwardly by the pressure loading module 300.

Here, the catch protrusions 212 are configured such that the bottom surfaces thereof are horizontal surfaces 212-1 to catch and hold the test strips 10 and the top surfaces thereof are inclined surfaces 212-2 inclined downwardly in the direction in which the catch protrusions 212 face each other.

According to this structure, as illustrated in FIG. 6, in the process of inputting the test strips 10 to the internal space of the internal container 200, the test strips 10 may be input by being pressed downwardly in the direction from the top to the bottom of the catch holders 210. In this case, the test strips input to the internal space of the internal container 200 are pressed upwardly by the pressure loading module 300 and are caught and held by the catch protrusions 212 of the catch holders 210. Thus, in the process of inputting the test strips 10 to the internal space of the internal container 200 using a separate gripping means (not shown), the gripping means does not hold or remains attached to the test strips 10 after the test strips 10 have been input. Accordingly, it is possible to prevent a problem in that the test strips in a stacked and arranged state are misaligned or overturned, so that a fabrication process of the strip cartridge can be performed more rapidly and accurately.

In addition, in the process of inputting the test strips 10 to the internal container 200 by pressing the test strips 10 downwardly in the direction of the top to the bottom of the catch holders 210, the inclined surfaces 212-2 provided on the top surfaces of the catch protrusions 212 of the catch holders 210 can facilitate the downward pressing and inputting of the test strips 10 to the internal container 200. Furthermore, since the catch holders 210 having the elastic bodies 211 are elastically deformable in the direction toward or away from each other, the elastic bodies 211 can be elastically deformed in the direction toward or away from each other while the test strips 10 are being input to the internal container 200 by downward pressing, thereby facilitating the inputting of the test strips 10.

In addition, the inner surfaces of the elastic bodies 211, facing each other, are provided as inclined surfaces 211-1 inclined toward each other in the direction from the bottom to the top, such that the test strips 10 are held and guided without movement in the internal space of the internal container 200.

According to the above-described structure, the test strips 10 stacked in the top-bottom direction in the internal space of the internal container 200 are loaded in the strip supply port 110 while being guided to a designated position. That is, in the integrated glucose meter, the discharge/supply means 20 discharges and supplies the test strips 10 individually to the outside, starting from the uppermost test strip 10, and the remaining test strips 10 stacked therebelow are moved upwardly by the elastic force of the pressure loading module 300. During the upward movement of the test strips 10 as described above, the test strips 10 are guided to a designated position by the inclined surfaces 211-1 of the elastic bodies 211. Accordingly, the test strips 10 can be supplied accurately.

In addition, a dehumidifier 400 may be provided inside of the main container 100. The dehumidifier 400 can remove moisture in the internal space in order to prevent degradations in the test strips 10.

The foregoing descriptions have been presented in order to explain certain principles of the present disclosure by way of example. A person skilled in the art to which the present disclosure relates could make various modifications and variations without departing from the essential features of the present disclosure. The foregoing embodiments disclosed herein shall be interpreted as being illustrative, while not being limitative, of the principle and scope of the present invention. It should be understood that the scope of the present invention shall be defined by the appended Claims.

## Claims

1. A strip cartridge for an integrated blood glucose meter, in which the strip cartridge is insertable into the integrated glucose meter and is configured such that a plurality of test strips (10) are stored therein, the strip cartridge comprising:
a vessel-shaped main container (100) having an open bottom end portion and a strip supply port (110) provided on a top end portion, the strip supply port (110) configured to allow test strips (10) to be discharged individually therethrough;
an internal container (200) shaped as a rectangular pipe adapted to allow test strips (10) to be stored and stacked on each other in a top-bottom direction in an internal space of the internal container (200), wherein the internal container (200) is adapted to be inserted into the main container (100) and includes catch holders (210) on a top end portion thereof to prevent test strips (10) from being dislodged upwardly;
a pressure loading module (300) coupled to a bottom end portion of the internal container (200) and configured to elastically press test strips (10) stacked in the internal space of the internal container (200), in a direction from bottom to top, **characterized in that** the catch holders (210) are configured such that an uppermost test strip (10) caught and held by the catch holders (210) is loaded in to the strip supply port (110) and wherein the catch holders (210) further include:
elastic bodies (211) protruding upwardly from opposite edges of the top end portion of the internal container (200) to face each other; and
catch protrusions (212) protruding from top end portions of the elastic bodies (211), respectively, in a direction in which the catch protrusions (212) face each other, and adapted to catch and hold test strips (10) pressed upwardly by the pressure loading module (300), wherein the elastic bodies (211) are elastically deformable in a direction toward or away from each other in order to input test strips (10) to the internal container (200) by pressing test strips (10) downwardly in the direction from the top to the bottom of the internal container (200).

2. The strip cartridge according to claim 1, wherein the catch protrusions (212) have horizontal bottom surfaces (212-1), by which test strips (10) are caught and held, and inclined top surfaces (212-2) inclined downwardly in a direction in which the catch protrusions (212) face each other.

3. The strip cartridge according to claim 2, wherein the elastic bodies (211) have inclined surfaces (211-1) facing each other, the inclined surfaces (211-1) being inclined toward each other in a direction from bottom to top, such that test strips (10) are held and guided without movement.

4. The strip cartridge according to claim 1, wherein the strip supply port (110) includes:
a protruding body (111) protruding upwardly from the top end portion of the main container (100), enabling a single test strip (10) to be loaded in an internal space of a protruding area; and
horizontal holes (112, 113) provided in opposite ends of the protruding body (111), enabling the single test strip (10) to be slid and discharged by being pressed by the separate discharge/supply means (20),
wherein an intermediate opening (114) is provided in an intermediate portion of the protruding body (111), such that the catch holders (210) of the internal container (200) are disposed in the intermediate portion of the protruding body (111).

5. The strip cartridge according to claim 4, wherein, among the horizontal holes(112, 113) provided in the opposite ends of the protruding body (111), a width of the horizontal hole (113), through which test strips (10) are discharged, is greater than a width of test strips (10), and a width of the remaining horizontal hole (112) is smaller than the width of test strips (10).

6. The strip cartridge according to one of claims 1 to 5, wherein the pressure loading module (300) includes:
a base body (310) coupled to the main container (100) to close the open bottom end portion of the main container (100);
a guide rod (320) coupled to the base body (310) to protrude upwardly so as to be inserted into the internal container (200);
a pressing body (330) coupled to the guide rod (320) to be movable linearly in a top-bottom direction; and
an elastic spring (340) applying upward elastic force to the pressing body (330) so that test strips (10) in the internal space of the internal container (200) are elastically pressed upward.

7. The strip cartridge according to one of claims 1 to 5, wherein a fixing guide (120) for fixing the position of the main container (100) inserted into the integrated glucose meter is provided on an outer surface portion of the main container (100).

## Patentansprüche

1. Streifenkartusche für ein integriertes Blutzuckermessgerät, wobei die Streifenkartusche in das integrierte Blutzuckermessgerät eingesetzt werden kann und konfiguriert ist, sodass eine Vielzahl von Teststreifen (10) darin gespeichert wird, die Streifenkartusche umfassend:
einen kesselförmigen Hauptbehälter (100), der einen offenen unteren Endabschnitt und einen Streifenzufuhranschluss (110), der an einem oberen Ende bereitgestellt ist, aufweist, wobei der Streifenzufuhranschluss (110) konfiguriert ist, um zu erlauben, dass Teststreifen (10) einzeln dort hindurch ausgegeben werden;
einen inneren Behälter (200), der als ein rechteckiges Rohr geformt ist, das angepasst ist, um zu erlauben, dass Teststreifen (10) in einem Innenraum des inneren Behälters (200) in einer Richtung von oben nach unten gespeichert und aufeinander gestapelt werden, wobei der innere Behälter (200) angepasst ist, um in den Hauptbehälter (100) eingesetzt zu werden, und Fanghalter (210) an einem oberen Endabschnitt davon beinhaltet, um zu verhindern, dass Teststreifen (10) nach oben verlagert werden;
ein Druckbelastungsmodul (300), das mit einem unteren Endabschnitt des inneren Behälters (200) gekoppelt und konfiguriert ist, um Teststreifen (10), die in dem Innenraum des inneren Behälters (200) gestapelt sind, in einer Richtung von unten nach oben elastisch zu drücken, **dadurch gekennzeichnet, dass** die Fanghalter (210) konfiguriert sind, sodass ein oberster Teststreifen (10), der von den Fanghaltern (210) gefangen und gehalten wird, in den Streifenzufuhranschluss (110) geladen wird, und wobei die Fanghalter (210) ferner Folgendes beinhalten:
elastische Körper (211), die von gegenüberliegenden Rändern des oberen Endabschnitts des inneren Behälters (200) nach oben hervorstehen und einander zugewandt sind; und
Fangvorsprünge (212), die jeweils von den oberen Endabschnitten der elastischen Körper (211) in einer Richtung hervorstehen, in der die Fangvorsprünge (212) einander zugewandt sind, und die angepasst sind, um Teststreifen (10) zu fangen und zu halten, die durch das Druckbelastungsmodul (300) nach oben gedrückt werden,
wobei die elastischen Körper (211) in einer Richtung aufeinander zu oder voneinander weg elastisch verformbar sind, um Teststreifen (10) in den inneren Behälter (200) einzuführen, indem Teststreifen (10) in der Richtung von oben nach unten des inneren Behälters (200) abwärts gedrückt werden.

2. Streifenkartusche nach Anspruch 1, wobei die Fangvorsprünge (212) horizontale Unterseiten (212-1), durch die Teststreifen (10) gefangen und gehalten werden, und geneigte Oberseiten (212-2), die in einer Richtung, in der die Fangvorsprünge (212) einander zugewandt, abwärts geneigt sind, aufweisen.

3. Streifenkartusche nach Anspruch 2, wobei die elastischen Körper (211) einander zugewandte geneigte Oberflächen (211-1) aufweisen, wobei die geneigten Oberflächen (211-1) in einer Richtung von unten nach oben zueinander geneigt sind, sodass die Teststreifen (10) ohne Bewegung gehalten und geführt werden.

4. Streifenkartusche nach Anspruch 1, wobei der Streifenzufuhranschluss (110) Folgendes beinhaltet:
einen hervorstehenden Körper (111), der von dem oberen Endabschnitt des Hauptbehälters (100) nach oben hervorsteht, um zu ermöglichen, dass ein einzelner Teststreifen (10) in einen Innenraum eines hervorstehenden Bereichs geladen wird; und
horizontale Löcher (112, 113), die in gegenüberliegenden Enden des hervorstehenden Körpers (111) bereitgestellt sind, die es ermöglichen, dass der einzelne Teststreifen (10) gleitet und ausgegeben wird, indem er von der separaten Entlade-/Zuführungseinrichtung (20) gedrückt wird, wobei eine zwischenliegende Öffnung (114) in einem zwischenliegenden Abschnitt des hervorstehenden Körpers (111) bereitgestellt ist, sodass die Fanghalter (210) des inneren Behälters (200) in dem zwischenliegenden Abschnitt des hervorstehenden Körpers (111) angeordnet sind.

5. Streifenkartusche nach Anspruch 4, wobei von den horizontalen Löchern (112, 113), die in den gegenüberliegenden Enden des hervorstehenden Körpers (111) bereitgestellt sind, eine Breite des horizontalen Lochs (113), durch das Teststreifen (10) ausgegeben werden, größer ist als eine Breite von Teststreifen (10), und eine Breite des verbleibenden horizontalen Lochs (112) kleiner ist als die Breite von Teststreifen (10).

6. Streifenkartusche nach einem der Ansprüche 1 bis 5, wobei das Druckbelastungsmodul (300) Folgendes beinhaltet:
einen Grundkörper (310), der mit dem Hauptbehälter (100) gekoppelt ist, um den offenen unteren Endabschnitt des Hauptbehälters (100) zu schließen;
eine Führungsstange (320), die mit dem Grundkörper (310) gekoppelt ist, um nach oben hervorzustehen, um so in den inneren Behälter (200) eingeführt zu werden;
einen Presskörper (330), der mit der Führungsstange (320) gekoppelt ist, um in einer Richtung von oben nach unten linear beweglich zu sein; und
eine elastische Feder (340), die eine nach oben gerichtete elastische Kraft auf den Presskörper (330) ausübt, sodass Teststreifen (10) in dem Innenraum des inneren Behälters (200) elastisch nach oben gedrückt werden.

7. Streifenkartusche nach einem der Ansprüche 1 bis 5, wobei eine Fixierführung (120) zum Fixieren der Position des in das integrierte Zuckermessgeräts eingesetzten Hauptbehälters (100) an einem äußeren Oberflächenabschnitt des Hauptbehälters (100) bereitgestellt ist.

## Revendications

1. Cartouche de bandelettes destinée à un glucomètre sanguin intégré, ladite cartouche de bandelettes pouvant être insérée dans le glucomètre intégré et étant conçue de sorte qu'une pluralité de bandelettes de test (10) y soient stockées, la cartouche de bandelettes comprenant :
un récipient principal (100) en forme de réservoir possédant une partie d'extrémité inférieure ouverte et un orifice d'alimentation de bandelettes (110) pourvu sur une partie d'extrémité supérieure, l'orifice d'alimentation de bandelettes (110) étant conçu pour permettre le déchargement individuel des bandelettes de test (10) à travers celui-ci ;
un récipient interne (200) en forme de tuyau rectangulaire étant adapté de manière à permettre le stockage des bandelettes de test (10) et leur empilement les unes sur les autres suivant la direction de haut en bas dans un espace interne du récipient interne (200), ledit récipient interne (200) étant adapté de manière à être inséré dans le récipient principal (100) et comportant des supports d'encliquetage (210) sur une partie d'extrémité supérieure de celui-ci pour empêcher le délogement vers le haut des bandelettes de test (10) ;
un module de chargement à compression (300) couplé à une partie d'extrémité inférieure du récipient interne (200) et conçu pour comprimer de manière élastique les bandelettes de test (10), empilées dans l'espace interne du récipient interne (200), suivant la direction allant du bas vers le haut,
**caractérisé en ce que** les supports d'encliquetage (210) sont conçus de sorte qu'une bandelette de test supérieure (10) encliquetée et maintenue par les supports d'encliquetage (210) soit chargée dans l'orifice d'alimentation de bandelettes (110) et lesdits supports d'encliquetage (210) comprenant en outre :
des corps élastiques (211) faisant saillie vers le haut depuis des bords opposés de la partie d'extrémité supérieure du récipient interne (200) pour se faire face l'un à l'autre ; et
des saillies d'encliquetage (212) faisant saillie à partir des parties d'extrémité supérieure des corps élastiques (211), respectivement, suivant une direction dans laquelle les saillies d'encliquetage (212) se font face l'une à l'autre, et adaptées de manière à encliqueter et à maintenir les bandelettes de test (10) comprimées vers le haut par le module de chargement à compression (300),
lesdits corps élastiques (211) étant déformables de manière élastique suivant une direction les rapprochant ou les éloignant l'un de l'autre afin d'introduire les bandelettes de test (10) dans le récipient interne (200) par compression des bandelettes de test (10) vers le bas suivant la direction allant du haut vers le bas du récipient interne (200).

2. Cartouche de bandelettes selon la revendication 1, lesdites saillies d'encliquetage (212) possédant des surfaces inférieures horizontales (212-1), au moyen desquelles les bandelettes de test (10) sont encliquetées et maintenues, et des surfaces supérieures inclinées (212-2) inclinées vers le bas suivant une direction dans laquelle les saillies d'encliquetage (212) se font face l'une à l'autre.

3. Cartouche de bandelettes selon la revendication 2, lesdits corps élastiques (211) comportant des surfaces inclinées (211-1) se faisant face l'une à l'autre, lesdites surfaces inclinées (211-1) étant inclinées l'une vers l'autre suivant la direction de bas en haut, de sorte que les bandelettes de test (10) soient maintenues et guidées sans mouvement.

4. Cartouche de bandelettes selon la revendication 1, ledit orifice d'alimentation de bandelettes (110) comprenant :
un corps saillant (111) faisant saillie vers le haut depuis la partie d'extrémité supérieure du récipient principal (100), qui permet le chargement d'une seule bandelette de test (10) dans un espace interne d'une zone saillante ; et
des trous horizontaux (112, 113) ménagés dans des extrémités opposées du corps saillant (111), qui permettent le coulissement de ladite seule bandelette de test (10) et son déchargement en étant comprimée par les moyens de déchargement/alimentation distincts (20),
une ouverture intermédiaire (114) étant ménagée dans une partie intermédiaire du corps saillant (111), de sorte que les supports d'encliquetage (210) du récipient interne (200) soient disposés dans la partie intermédiaire du corps saillant (111).

5. Cartouche de bandelettes selon la revendication 4, parmi les trous horizontaux (112, 113) ménagés dans les extrémités opposées du corps saillant (111), la largeur du trou horizontal (113), à travers laquelle les bandelettes de test (10) sont déchargée, étant supérieure à la largeur des bandes de test (10), et la largeur du trou horizontal restant (112) étant inférieure à la largeur des bandes de test (10).

6. Cartouche de bandelettes selon l'une des revendications 1 à 5, ledit module de chargement à compression (300) comprenant :
un corps de base (310) couplé au récipient principal (100) destiné à fermer la partie d'extrémité inférieure ouverte du récipient principal (100) ;
une tige de guidage (320) couplée au corps de base (310) destinée à faire saillie vers le haut afin d'être insérée dans le récipient interne (200) ;
un corps de compression (330) couplé à la tige de guidage (320) destiné à être mobile linéairement suivant la direction de haut en bas ; et
un ressort élastique (340) appliquant une force élastique vers le haut sur le corps de compression (330) de sorte que les bandelettes de test (10) dans l'espace interne du récipient interne (200) soient comprimées de manière élastique vers le haut.

7. Cartouche de bandelettes selon l'une des revendications 1 à 5, un guide de fixation (120) destiné à fixer la position du récipient principal (100) inséré dans le glucomètre sanguin intégré étant pourvu sur une partie de surface externe du récipient principal (100).
